# EUROPEAN PATENT APPLICATION

(11) **EP 0 933 355 A1**
(43) Date of publication of application: **04.08.1999**
(21) Application number: 97204097.6
(22) Date of filing: 24.12.1997
(51) Int. Cl.: C07C 213/00, C07C 215/52, C07J 41/00, G01N 33/58

(54) **Preparation of tyramide conjugates**

(71) Applicant: Universiteit Maastricht, 6229 ER Maastricht (NL)
(72) Inventor: Hopman, Anthonius Hendrikus Nicolaas, 6245 HD Eijsden (NL); Speel, Ernst Jan Maria, 8916 Jonen (CH); Ramaekers, Franciscus Charles Servatius, 6213 CB Maastricht (NL)
(74) Representative: Hoogstraten, Willem Cornelis Roeland

(57) **Abstract**

A method for the preparation of tyramide conjugates is provided which can be utilized in the CARD amplification system. The method comprises reacting a suitable ester of a labeling compound with tyramine in a substantially water-free organic solvent at a constant pH of about 7 to about 9.5, preferably from 7.0 to 8.0. The reaction is suitably carried out in a buffered medium. Preferably, the NHS ester is applied of biotin, digoxigenin, dinitrobenzene, trinitrobenzene or the fluorochromes fluorescein, rhodamine, cyanine, coumarin and BODIPY dyes. The invention provides an easy and fast method to prepare a variety of known and new tyramide conjugates in bulk amounts at high purity and at relatively low cost. The tyramide conjugates thus obtained can be suitably applied in affinity assays including immunohistochemistry and *in situ* hybridization ISH procedures to detect with high efficiency protein epitopes or repetitive and single-copy DNA target sequences in cell preparations.

## Description

### Field of the Invention

The present invention is in the field of diagnostics, especially immunoassays. More in particular, the present invention relates to a new and efficient method of preparing various tyramide conjugates, which can be used in the catalyzed reporter deposition ("CARD") amplification system.

### Background of the Invention and Prior Art

In 1989, Bobrow *et al*. introduced an alternative for solid-phase systems to improve sensitivity in immuno and DNA/RNA probe assays, which was termed Catalyzed Reporter Deposition or CARD. The system is based upon the use of the immobilized reporter enzyme peroxidase to catalyze the deposition of multiple copies of a detectable label onto tissue sections or cell preparation surfaces blocked with protein. Detectable labels proposed by Bobrow *et al*. are phenolic based labeled substrates such as biotinyl-tyramide and fluorescyltyramide. The reaction is quick (less than 10 minutes) and results in a covalent biotin or fluorochrome deposition right at the enzyme site with minimal loss in resolution.

The use of CARD as a means of signal amplification was successfully implemented as a detection system in several molecular procedures, such as ELISA, Western blotting, immunocytochemistry, and *in situ* hybridization (ISH). See Bobrow *et al*. 1991; Adams 1992; Berghorn *et al*. 1994; Kerstens *et al*. 1995; Raap *et al*. 1995; Van Heusden *et al*. 1996; Van Gijlswijk *et al*. 1997.

However, sofar only a few references describe the preparation of tyramide conjugates. For example, Bobrow *et al*. (1991) and U.S. Patent No. 5,196,306 disclose the preparation of "biotine-tyramine" by either mixing a solution of tyramine in dimethylsulfoxide (DMSO) with an excess of a solution of biotin-N-hydroxysuccinimide (NHS) ester in DMSO overnight at room temperature, or by treating a solution of an equimolar amount of biotin-NHS and tyramine in dimethylformamide (DMF) with triethylammonium bicarbonate, pH 7.5, and then heating at 50°C for 3 hours. Adams (1992) and Kerstens *et al*. (1995) prepared biotinyl-tyramide by dissolving the active ester and tyramine in an aqueous borate buffer (pH 8.0) resulting in a slightly cloudy solution. Raap *et al*. (1995) prepared three fluorochrome-labeled tyramides by mixing tyramine in DMSO with an excess of NHS esters of the fluorochromes.

All methods mentioned above suffer from certain drawbacks. For example, cloudiness is an indication that the reaction components are not properly dissolved and irreproducible results may be expected. The same applies when the reaction medium is not buffered, since it has appeared that the effectiveness of the coupling reaction strongly depends *inter alia* on the pH of the reaction medium. If the reaction is carried out in the presence of water, undesired hydrolysis may easily occur.

It is further clear from the prior art that only a limited number of tyramide conjugates is available today which hampers their application in multiple target analyses. Therefore, there is still a need for new tyramide conjugates and for a rapid and easy method of preparing large quantities of such conjugates.

### Summary of the Invention

The present invention provides in one aspect a method for the preparation of tyramide conjugates which can be utilized in the CARD amplification system. The method comprises reacting a suitable ester of a labeling compound with tyramine or a salt thereof in a substantially water-free organic solvent at a pH of about 7 to about 9.5, preferably from 7.0 to 8.0.

According to a preferred embodiment of the invention the reaction is carried out at constant pH, preferably by using a buffered medium. A suitable and preferred solvent is dimethylformamide. The pH is suitably adjusted with an organic base, for example triethylamine or a similar compound.

According to another aspect of the invention the ester of the labeling compound is the succinimidyl ester of a compound selected from the group of biotin, digoxigenin, dinitrobenzene, trinitrobenzene and the fluorochromes fluorescein, rhodamine, cyanine, coumarin and BODIPY dyes, and structurally closely related compounds.

In still a further aspect, the invention provides new tyramide conjugates which include digoxigenin-tyramide and 2,4,6-trinitrophenyl-tyramide.

The invention provides an easy and fast method to prepare a variety of known and new tyramide conjugates in bulk amounts at high purity and at relatively low cost. The tyramide conjugates thus obtained can be suitably applied in single and multiple target immunocytochemistry and *in situ* hybridization ISH procedures to detect with high efficiency epitopes or both repetitive and single-copy DNA target sequences in cell preparations.

Accordingly, in still a furter aspect of the invention affinity-assays are provided which may be in the form of a kit, including ELISA, immunoblotting, immunocytochemistry and ISH, comprising a tyramide conjugate as disclosed and claimedin the present invention.

These and other aspects will be further described in the following specification.

### Brief description of the drawings

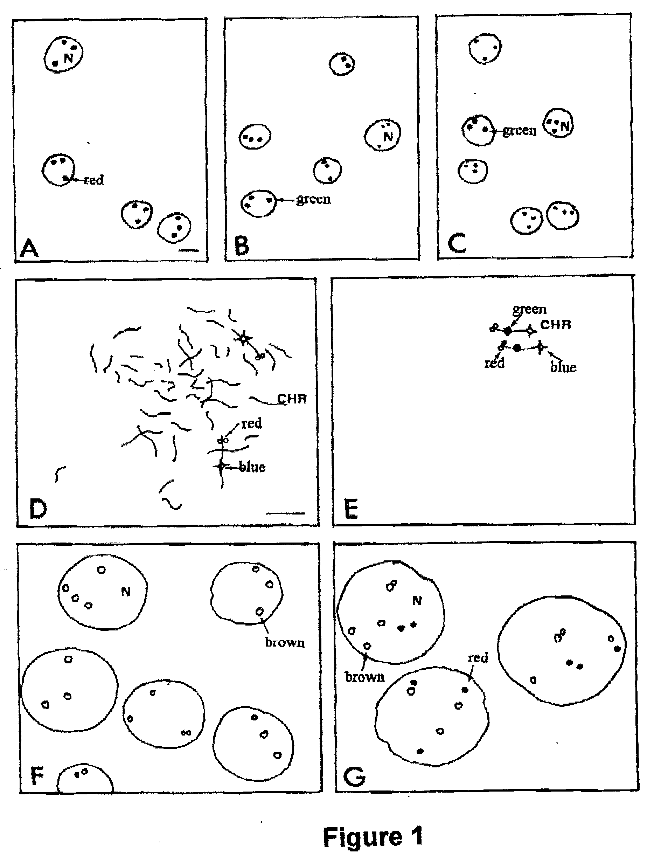

Figure 1 is a schematic representation of seven micro color photographs (a-g) which show fluorescence (a-e) and brightfield ISH results after CARD detection of various DNA probes in T24 cells (a-c,f,g) and chromosome preparations (d,e). N is nucleus and CHR stands for chromosome.

The results were obtained without DNA counterstaining (e) or by counterstaining with either DAPI (a), PI (b,c), YOYO (d) or hematoxylin (f,g).
Figures 1 (a-c) show the detection of the chromosome region 1q12 (biotin) with peroxidase conjugated avidin ("AV-PO") followed by Cy3-tyramide (a; red); trinitrophenyl-tyramide ("TNP-tyramide") followed by rabbit anti-DNP ("RADNP"; DNP = dinitrophenyl) and fluorescein conjugated rabbit anti-digoxigenin ("SWAR-FITC") (b; green); and digoxigenin-tyramide ("DIG-tyramide") followed by fluorescein conjugated rabbit anti-digoxigenin ("RADIG-FITC") (c; green).
Figure 1(d) shows the simultaneous detection of 1q12 (biotin) and 1p36 (digoxigenin) with, consecutively, peroxidase conjugated sheep anti-digoxigenin ("SHADIG-PO") and Cy3-tyramide (red), and AV-PO combined with aminomethyl coumarin-tyramide ("AMCA-tyramide") (blue).
Figure 1(e) shows the simultaneous detection of chromosome 1p36 (biotin), 1q42-43 (digoxigenin) and 1q12 (FITC) regions detected with consecutively AV-PO and aminomethyl coumarin-tyramide (blue), SHADIG-PO and rhodamine-tyramide ("RHO-tyramide") (red), and peroxidase conjugated mouse anti-FITC ("MAFITC-PO") with fluorescein-tyramide (green), respectively.
Figure 1(f) shows the detection of the chromosome 4p16 region (digoxigenin) with SHADIG-PO, digoxigenin-tyramide ("DIG-tyramide"), SHADIG-PO and diaminobenzidine ("DAB") reaction (brown).
Figure 1(g) shows the simultaneous detection of the chromosome 1q12 (digoxigenin) and 1p36 (biotin), detected with SHADIG-APase/Fast Red (red) and AV-PO, biotine-tyramide ("BIO-tyramide"), AV-PO/DAB (brown), respectively. SHADIG-APase is alkaline phosphatase conjugated sheep anti-digoxigenin.

Bar = 5 µm; images with the same magnification a-c; d-f.

### Detailed Description of the Invention

The term "tyramine" as used in the present specification is meant to indicate tyramine and structurally and functionally closely related compounds and derivates thereof. Tyramine salt includes any convenient organic and inorganic salt of said tyramine. A preferred salt is tyramine.HCl.

According to the present invention a tyramide conjugate is prepared by reacting a suitable ester of a labeling compound with tyramine or a salt thereof in a substantially water-free organic solvent at a pH of about 7 to about 9.5, preferably from 7.0 to 8.0. The reaction is preferably carried out at constant pH, more preferably by using a buffered medium. A suitable and preferred solvent is dimethylformamide. The pH is suitably adjusted with an organic base, for example triethylamine or a similar compound.

The ester of the labeling compound is preferably the N-hydroxysuccinimidyl (NHS) ester of a reactive compound suitably selected from the group of biotin, digoxigenin, dinitrobenzene, trinitrobenzene and the fluorochromes fluorescein, rhodamine, cyanine, coumarin and BODIPY dyes, and structurally closely related compounds. Thus, the resulting esters are, for example, biotin-NHS, digoxigenin-NHS, dinitrophenyl-NHS, trinitrophenyl-NHS, fluorescein-NHS, rhodamine-NHS, cyanine-NHS, coumarin-NHS, BODIPY-NHS, etc.

The above-mentioned fluorochromes are in most cases groups of compounds. It is understood that each and any individual compound of these groups, whether or not explicitly mentioned in this specification, is within the scope of the present invention. Such individual compounds are, for example, 7-amino-4-methylcoumarin, Cy2, Cy3, Cy5, Cy5.5, BODIPY FL-X,SE, BODIPY TR-X,SE, and esters and tyramide conjugates thereof.

The NHS and other esters of the labled compounds defined above are partly known and described in the literature. Otherwise they can be prepared in a manner known in the art for the preparation of similar or related compounds.

The organic solvent is preferably water-free DMF, or the like. The tyramine is preferably dissolved in the organic solvent prior to the coupling reaction and an organic base is added to deprotonate the amino group of the tyramines (or tyramine.HCl). A suitable base is triethylamine (TEA) which is added in an amount to adjust the pH to the desired value which is generally between about 7 and about 9.5, preferably from 7.0 to 8.0.

The hydrolysis of the active fluorochrome or hapten N-hydroxysuccinimide esters is avoided by performing the reaction in a water-free medium and by adding an organic base to deprotonate the amino group of tyramine. Preferably, TEA or a similar compound is used as the base. The amount of base is preferably in the range of about 1.0 to 1.5 molar relative to the tyramine.HCl. Further addition of base may result in some hydrolysis of the active fluorochrome or hapten NHS esters. The reaction of the labeling compound and tyramine is conveniently carried out with about equimolar amounts of the reactants.

Furthermore, according to the method of the invention the reactants are completely dissolved in the organic solvent, which is preferably DMF. The reaction time is usually in the range of 0.5 h to several hours at room temperature (20°C), preferably 1-2 hours depending on the reactivity of agents used. This is relatively short as compared to the prior art methods (Bobrow *et al.* 1991; Adams 1992; Raap *et al.* 1995; Kerstens *et al*. 1995). The reaction is preferably carried out at room temperature.

According to a further aspect of the invention 2,4,6-trinitrophenyl (TNP)-tyramide is provided. This compound can be prepared in substantially the same way as described above, e.g. by reacting a reactive ester of 2,4,6,-trinitrobenzenesulfonic acid with tyramine. However, in this case a preferred method of preparation is adding a reactive 2,4,6-trinitrobenzene derivative, e.g. 2,4,6-trinitrobenzensulfonic acid, to tyramine which is dissolved in either MilliQ/DMF basified with TEA or in an aqueous buffer solution at about pH 9.5 (e.g. with NaHCO₃ buffer), followed by precipitation of the TNP-tyramide.

Likewise, a preferred method of preparing dinitrophenyl (DNP)-tyramide, and in particular 2,4-dinitrophenyl-tyramide, is using the corresponding dinitrofluorobenzene as the starting compound.

The preparation of tyramide conjugates according to the invention results in products that can be successfully applied in the CARD amplification step for ISH.

The tyramide conjugates which are prepared by the method according to the present invention are recovered by methods known in the art. The tyramide conjugates are usually obtained in substantially pure form and can be applied as such. If desired, further purification steps can be performed which are well within the reach of the man skilled in the art.

The method of preparing tyramide conjugates according to the present invention is suitable for bulk production. In particular TNP-tyramide can be produced in gram quantities with high purity, opening the possibility to use this tyramide in a routine setting on a daily basis. In diagnostic pathology as well as other immunochemical studies this means a significant reduction in the costs for primary antibodies, since these reagents can be diluted several magnitudes more.

The applicability of the newly synthesized tyramides was successfully tested in single- and multiple-target ISH procedures visualizing both repetitive and single-copy target sequences in cell preparations with CARD amplification fluorescence- or brightfield detection systems.

The tyramide conjugates were applied in model systems to test the sensitivity of immunocytochemical amplification systems. All synthesized products could be applied within at least the same concentration range as described in the literature, indicating the efficiency of the method of preparation. The applicability and sensitivity of the haptenized tyramides in brightfield ISH is illustrated in Figures 1f and g. The intense signals for the cosmid probes illustrate the sensitivity and is comparable with the results obtained by Kerstens *et al*., 1995. The large size of the ISH signal for the 1p36 target as compared to the 1q12 target, is the result of diffusion of the tyramides during the PO/tyramide reaction, followed by the PO/DAB reaction.

In conclusion, the method of preparing tyramide conjugates according to the present invention is an easy one, allowing to prepare a variety of new substrates for peroxidase cytochemistry since many active esters of fluorochromes or other haptens are commerically available. The protocol minimizes problems arising from dissolving of these active esters and facilitates the coupling reaction.

The invention is further illustrated by the following experimental work, which however should not be interpreted as limiting the scope of the invention in any way.

### MATERIALS AND METHODS

### Reagents for the preparation of tyramide conjugates

The following N-hydroxysuccinimide (NHS) esters were purchased from Pierce, Rockford, IL, USA:
a) biotin-NHS [sulfosuccinimidyl-6-(biotinimide)hexanoate], "BIO-NHS" (MW: 557);
b) fluorescein-NHS [fluorescein, succinimidyl ester], "FLU-NHS" (MW: 473];
c) rhodamine-NHS [5-(and 6)-carboxyfluorescein, succinimidyl ester], "RHO-NHS" (MW: 527), and
d) 7-amino-4-methylcoumarin-3-acetic acid, succinimidyl ester; "AMCA-NHS", MW: 330.
   The NHS esters of:
e) digoxigenin [(digoxigenin-3-O-succinyl-ε-aminocaproic acid, succinimide ester], "DIG-NHS", MW: 659; and
f) Cy3 [Cy3, succinimidyl ester], "Cy3-NHS", MW: 766; one vial contains 200 µg);
were purchased from Boehringer Mannheim, Germany and Amersham Life Science, Arlington Heights, IL, USA, respectively.

Tyramine.HCl (MW: 173) was purchased from Sigma Chemical Compounds, St. Louis, MO, USA, and dimethylformamide (DMF; water free) and triethylamine (TEA; 7.2 M) were purchased from Pierce.

### Tyramide synthesis in organic media

Table 1 summarizes the various amounts of active esters (X-NHS), tyramine, and TEA which were reacted in accordance with the present invention to prepare tyramide conjugates in a one-step synthesis. The NHS esters are prone to hydrolysis and are light-sensitive. For this reason the active esters were freshly dissolved only shortly before use. All esters were dissolved in DMF at concentrations as indicated in Table 1 to obtain an active ester stock solution "A" (10 mg per ml DMF). The tyramine stock solution "B" was prepared by dissolving 10 mg per ml DMF (equivalent to 58 µmol) to which a 1.25 times equimolar amount of TEA (10 µl of 7.2 M stock solution; 72.5 µmol) was added. For an efficient acylation, the active esters (stock solution A) were added in 1.1 times equimolar amounts to the tyramine stock solution B and left at room temperature in the dark for 2 hr. The tyramide conjugates obtained were diluted with ethanol to obtain stock solutions "C" of 1 mg per ml. The tyramide stock solutions could be safely stored for at least 8 months at 4°C without any reduction in reactivity.

### TNP-tyramide synthesis in aqueous media

TNP-tyr was prepared in two different ways: 1) by mixing equimolar amounts of 2,4,6-trinitrobenzenesulfonic acid (TNBS; Pierce; MW 690, 5% solution in water; Pierce) and tyramine with an excess TEA to obtain a pH of 9.5. The reaction was performed for 2 hr at room temperature; 2) by mixing equimolar amounts of TNBS (20 mg/400 µl) and tyramine dissolved in 0.3 M NaHCO₃ buffer of pH 9.5. This reaction was performed for 1 hr in the dark at room temperature in a 10 ml plastic tube, with mixing on a vortex every 15 min. Already after the first 15 min a precipitate of TNP-tyr was formed. To complete the reaction the mixture was left at room temperature for 1 hr. The precipitate was pelleted, the supernatant removed and washed subsequently with the carbonate buffer and MilliQ. The pellet was dried *in vacuo* and dissolved in 0.2 ml DMF to which 0.8 ml ethanol was added.

### Cell processing

A 70% ethanol suspension of the human bladder carcinoma cell culture T24 (Bubenik *et al*., 1973) (DNA index 1.6; trisomic for 1q12, tetrasomic for 1p36, and trisomic for 4p16) and methanol:acetic acid (3:1) fixed human lymphocyte chromosome spreads were pretreated by pepsin digestion to improve DNA probe and conjugate penetration, as previously described (Hopman *et al*., 1988; Speel *et al*., 1993).

### DNA probes and labeling procedures

The DNA probes for chromosome 1q12 (pUC 1.77), 1p36 (p1-79), 1q42-43 (pH5SB), 4p16 (c5.5) have been described by Cooke and Hindley (1979), Buroker *et al*. (1987), Little and Braaten (1989) and Landegent *et al*. (1986), respectively. The DNA probes were labeled with biotin-11-dUTP (Enzo Diagnostics, New York, NY), digoxigenin-11-dUTP, or fluorescein-12-dUTP (Boehringer Mannheim) in a standard nick-translation reaction and used in single-, double-, or triple-target ISH procedures.

### ISH procedure

The DNA probes described above were used at a concentration of 0.4 ng/µl (pUC 1.77), 1 ng/µl (p1-79) or 5 ng/µl (pH5SB and c5.5) and hybridized in different combinations in a hybridization buffer containing 50% formamide, 2xSSC (0.3 M NaCl, 30 mM Na-citrate) pH 5.0, 10% dextran sulphate, 0.2 µg/µl herring sperm DNA as carrier DNA, and 0.2 µg/µl yeast tRNA as carrier RNA. An excess of total human placenta DNA was added as competitor DNA when c5.5 (100 x excess competitor) or pH5SB (250 x excess competitor) were included. Ten µl hybridization buffer was added to each slide under a coverslip (20x20 mm). Denaturation was performed on the bottom of a metal box in a water bath at 70°C for 3 min and hybridization was performed overnight at 37°C. The slides were washed twice for 5 mitt at 42°C with 2xSSC, containing 0.05% Tween 20, followed by two 5-min washes at 60°C 0.1xSSC and a 5-min wash with 4xSSC, pH 7.0, containing 0.05% Tween 20 (Buffer D) at room temperature.

### Cytochemical detection procedures

To reduce background staining in the cytochemical detection procedures, the slides were pre-incubated for 10 min at 37°C with 4xSSC, pH 7.0, containing 5% non-fat dry milk (Buffer E), followed by dipping in Buffer D (see above). For all the detection procedures, the avidin conjugates were diluted in Buffer E, and all the antibody conjugates were diluted in PBS containing 0.05% Tween 20 (Buffer F) and 2% normal goat serum (NGS). After each incubation step of 20-30 min at 37°C, the slides were rinsed twice in Buffer D in case of avidin conjugates (ABC system) or Buffer F in case of antibody conjugates.

### Single-target ISH

The biotinylated pUC 1.77 probe was detected by peroxidase (PO)-conjugated avidin (AV-PO, 1:50 dilution; DAKO), followed by CARD amplification as previously described (Speel *et al*. 1997). After cytochemical detection of the probes, the different tyramide conjugates RHO-tyr: 1:1000; BIO-tyr, DIG-try : 1:500; TNP-tyr, AMCA-tyr, FLU-try, Cy3-tyr 1:250 (diluted from the 1 mg/ml stock solution C with concentrations of 1.8, 1.7, 1.5, 1.4, 3.0, 2.0, and 1.3 mM, respectively) were applied in PBS containing 0.1 M imidazole, pH 7.6, and 0.001% H₂0₂ (50 µl under a coverslip) for 5-10 min at 37°C. Thereafter the slides were washed twice with buffer F.

The tyramide conjugates were detected as follows: TNP-tyramide was detected with rabbit anti-DNP (RADNP, 1:100, DAKO) and FITC-labeled Swine anti-Rabbit IgG (SWAR-FITC, 1:80, DAKO); DIG-tyramide with FITC-labeled rabbit anti-DIG (RADIG-FITC, 1:100, Boehringer); BIO-tyramide with FITC-labeled Avidin (AV-FITC, 1:500, Vector, Brunschwig Chemie, Amsterdam, The Netherlands). The digoxigenin-labeled c5.5 probe was visualized by peroxidase (PO)-conjugated sheep anti-DIG Fab fragments (SHADIG-PO, 1:100; Boehringer), followed by CARD amplification with DIG-tyramides (see above), SHADIG-PO and the PO/DAB reaction (see above).

### Multiple-target ISH

For multiple-target ISH with fluorochrome-labeled tyramides, the CARD detection of the various probes was performed sequentially, with a 0.01 M HCl treatment for 10 min at room temperature between the individual detection steps to inactivate PO activity (Speel *et al*. 1994, 1995). Labeled probes were detected as follows: BIO with AV-PO, and optionally followed by biotinylated goat anti-Avidin (GAA-BIO) and AV-PO; DIG with SHADIG-PO or mouse anti-DIG (MADIG) and PO conjugated rabbit anti-mouse (RAM-PO); FITC with PO conjugated anti-FITC (AFITC-PO, 1:200; NEN Life Science Products, Boston, MA). In all cases cytochemical detection was followed by CARD amplification with fluorochrome-labeled tyramides. For multiple-target brightfield ISH, the DIG-labeled pUC1.77 probe was detected by alkaline phosphatase (APase)-conjugated sheep anti-DIG Fab fragments (SHADIG-APase; 1:100; Boehringer Mannheim, Germany) and the APase-Fast Red reaction (see below), followed by visualization of the biotinylated 1p36 probe with AV-PO, CARD amplification with BIO-tyramide (see below), AV-PO and the PO/DAB reaction (see below).

### PO/DAB and APase/Fast Red reactions

PO/DAB reaction (brown precipitate; Graham and Karnovsky, 1966). Before use 1 ml 3,3 diaminobenzidine tetrachloride (DAB; Sigma) in PBS (5 mg/ml stock) was mixed with 9 ml 0.1 M imidazole in PBS pH 7.6, and 10 µg 30% H₂O₂. The slides were incubated with 0.1-1.0 ml for 5-15 mitt at 37°C and washed 3x5 mitt with PBS.

APase/Fast Red reaction (red precipitate; Speel *et al*., 1993). Before use 4 ml 0.2 M Tris-HCl, pH 8.5, containing 10 mM MgCl₂ and 5% polyvinyl alcohol (PVA, Mw 40,000; Sigma) were mixed with 250 µg of this buffer without PVA containing 1 mg naphthol-ASMX-phosphate (Sigma), and 750 µg buffer without PVA containing 5 mg Fast Red and TR salt (Sigma). The slides were incubated with 0.1-1.0 ml reaction mixture for 5-15 min at 37°C and washed 3x5 min with PBS. Counterstaining was performed with hematoxylin, the slides then optionally dehydrated and mounted with PBS/glycerol (1:9 v/v).

### Microscopy

Microphotographs were made on a Leica DM RBE microscope equipped with the Metasystem Image Pro System (black and white CCD camera, Sandhausen, Germany). Images were captured using the ISIS program for fluorescence *in situ* hybridization (FISH) and for brightfield ISH additional green, red and blue filters were applied. Images were processed in Adobe Photoshop 3.0 at a resolution of 120 pixels/inch.

### EXAMPLES

### Preparation of tyramide conjugates

Table 1 summarizes the preparation of seven tyramide conjugates. For this purpose a 1.1 times molar excess of the active hapten- (BIO, DIG) or fluorochrome- (RHO, FLU, Cy3, AMCA) succinimidyl esters, freshly dissolved in DMF, were added to the tyramine. The tyramine was also dissolved in DMF adjusted with TEA to obtain a pH between 7.0 - 8.0. After a 2 hr reaction the synthesized tyramide conjugates were diluted to a concentration of 1 mg/ml by adding ethanol. These stock solutions were further diluted to working concentrations, in the range of 1-10 µM, and the tyramide conjugates were used without further purification for ISH probe detection (see below).

**Table 1**

| Reaction conditions for the one-step preparation of various tyramide conjugates | | | | |
|---|---|---|---|---|
| activated conjugates^{a} | amount (mg) | Stock A active ester in DMF(µl) | Stock B tyramine^{b} (µl) | Stock C µl ethanol added to obtain labeled tyramide concentration of 1 mg/ml |
| BIO-NHS | 1 | 100 | 28.4 | 871.6 |
| DIG-NHS | 1 | 100 | 24 | 876 |
| Cy3-NHS | 0.2 | 80 | 4.1 | 115.9 |
| FLU-NHS | 1 | 100 | 33.4 | 866.6 |
| RHO-NHS | 1 | 100 | 30 | 870 |
| AMCA-NHS | 1 | 100 | 48 | 852 |
| TNBS^{c} | 1 | 22.5^{c} | 25 | 952.5 |

| | | | | |
|---|---|---|---|---|
| ^{a} All active esters were dissolved in dimethylformamide (DMF). Abbreviations used: BIO: biotin, DIG: digoxigenin, Cy3: cyanine 3, FLU: fluorescein, RHO:tetramethylrhodamine, AMCA: aminomethyl coumarin acetic acid, NHS: N-hydroxysuccinimide ester. | | | | |
| ^{b} Tyramine-HCl was dissolved at 10 mg/ml in dimethylformamide and to this solution a 1.25 times equivalent amount of triethylamine (10 µl of 7.2 M) per ml was added (final pH: 7 < pH < 8). | | | | |
| ^{c} TNBS: 2,4,6,-trinitrobenzenesulfonic acid (5% stock solution in Milli Q). To 20 µl TNBS stock solution 2.5 µl extra TEA was added to obtain a pH of 9.5. | | | | |

TNP-tyramide was prepared by mixing an aqueous TNBS solution with tyramine/TEA (see above) or in 0.3 M NaHCO₃ buffer pH 9.5. Since TNBS is not prone to hydrolysis in water, the coupling reaction can be performed with an excess of base in both organic and aqueous reaction media. During the reaction of TNBS with tyramine in 0.3 M NaHCO₃ buffer, the produced TNP-tyramide precipitated spontaneously, since these molecules are only slightly water soluble. This precipitation reaction therefore allows the easy isolation of a pure reaction product while the unreacted tyramine and TNBS remain in solution. In this way large quantities of this conjugate can be produced and isolated. The TNP-tyramide is dissolved in DMF and diluted with ethanol.

### Application and sensitivity of tyramide conjugates in fluorescence ISH

All tyramide conjugates produced according to the present invention were tested in single-target ISH experiments on T24 cells, using the biotinylated probe for 1q12 and one detection layer of AV-PO. The fluorochrome-labeled tyramides (Cy3, FLU, RHO and AMCA) were deposited in CARD amplification reactions for 5 min at 37°C and could be visualized directly in the fluorescence microscope. T24 cells displayed 3 ISH signals per nucleus in all cases, as shown in Figure 1a for Cy3-tyramide. Depositions of the hapten-labeled tyramides (BIO, DIG and TNP) required additional incubations with fluorescent detection conjugates to visualize them in the fluorescence microscope. For example, Figures 1b and c show the detection of chromosome 1q12 with TNP-tyramide/RADNP/SWAR-FITC and DIG-tyramide/SHADIG-FITC, respectively. All the tyramides could be applied at concentrations of 1-10 µM in a 5-10 min amplification reaction at 37°C in PBS/imidazole. The RHO-tyramide appeared to be the most sensitive conjugate as was to be expected on basis of previously described observations (Raap *et al*. 1995, Speel *et al*. 1997).

The applied tyramide conjugate concentrations were compared to the conditions recommended in the commercially available kits (BLAST, TSA Renaissance systems or C.S.A. kits) and with the concentrations known from the literature (Raap *et al*. 1995, Chao *et al*. 1996, Van Gijlswijk *et al*. 1997, Speel *et al*. 1997). No differences were found when utilizing the commercially available tyramide conjugates for localizing the 1q12 targets in T24 nuclei. For the new DIG- and TNP-tyramides no reference values are available but their optimal concentrations were within the same range as the other tyramide conjugates.

The obtained fluorochrome-conjugated tyramides were also tested in multiple-target FISH procedures. Figure 1d shows a double-target FISH using AMCA-tyramide and Cy3-tyramide. The chromosomal targets 1q12 and 1p36 were detected, subsequently, with SHADIG-PO/Cy3-tyramide and AV-PO/AMCA-tyramide resulting in discrete CARD signals. For this purpose the PO activity remaining after the first CARD reaction step was inactivated by a mild acid incubation step, before application of the next CARD detection step. In this way also a triple-target fluorescence ISH experiment with CARD detection could be performed. Figure 1e shows the localization of the 1q42-43, 1q12 and 1p36 targets on human chromosome 1 with AMCA-tyramide, RHO-tyramide and FLU-tyramide, respectively. The chromosomes were not counterstained to avoid color overlap of the general DNA stains (DAPI or PI) with one of the CARD signals.

### CARD amplification in brightfield ISH

The hapten-tyramides could also be applied to localize both repetitive and single-copy gene DNA sequences in brightfield ISH. As an example, Figure 1f shows the detection of the digoxigenin labeled c5.5 cosmid-probe with SHADIG-PO, DIG-tyramide, SHADIG-PO and the PO/DAB reaction. In the T24 interphase nuclei both three and four ISH signals per nucleus could be visualized, as was expected from earlier unpublished results using fluorescence ISH (78% trisomy, 8% tetrasomy). Double-target brightfield ISH was carried out to detect 1q12 with APase/Fast Red, after which the 1p36 target could be is detected using the BIO-tyramide. The latter target was detected in PO/DAB (Figure 1g).

### REFERENCES

Adams J (1992) Biotin amplification of biotin and horseradish peroxidase signals in histochemical stains. J Histochem Cytochem 40:1457-1463.

Berghorn KA, Bonnett JH, Hoffman GE (1994) cFos immunoreactivity is enhanced with biotin amplification. J Histochem Cytochem 42:1635-1642

Bobrow MN, Shaughnessy KJ, Litt GJ (1991) Catalyzed reporter deposition, a novel method of signal amplification. J Immunol Methods 137:103-112.

Bobrow MN, Harris TD, Shaughnessy KJ, Litt GJ (1989) Catalyzed reporter deposition, a novel method of signal amplification. J Immunol Methods 125:279-285.

Bubenik J, Baresová M, Viklicky V, Jakoubková J, Sainerová H, Donner J (1973) Established cell line of urinary bladder carcinoma (T24) containing tumour-specific antigen. Int J Cancer 11:765-773.

Buroker NR, Bestwick R, Haight G, Magenis RE, Litt (1987) A hypervariable repeated sequence on human chromosome 1p36. Hum Genet 77:175-181.

Chao J, DeBiasio R, Zhu Z, Giuliano KA, Schmidt BF (1996) Immunofluorescence signal amplification by the enzyme-catalyzed deposition of a fluorescent reporter substrate (CARD). Cytometry 23:48-53.

Cooke JH, Handle J (1979) Cloning of human satellite III DNA: different components are on different chromosomes. Nucleic Acids Res 6:3177-3197.

Graham RC, Karnovsky MJ (1966) The early stages of absorption of injected horseradish peroxidase in the proximal tubules of mouse kidney: ultrastructural cytochemistry by a new technique. J Histochem Cytochem 14:291-302.

Hopman AHN, Wiegant J, Tesser GI, Van Duijn P (1986a) A non-radioactive in situ hybridization method based on mercurated nucleic acid probes and sulfhydryl-hapten ligands. Nucleic Acid Res 14:6471-6488.

Hopman AHN, Wiegant J, Raap AK, Landegent JE, Van der Ploeg M, Van Duijn P (1986b) Bi-color detection of two target DNAs by non-radioactive in situ hybridization. Histochemistry 85:1-4.

Hopman AHN, Ramaekers FCS, Raap AK, Beck JLM, Devilee P, Van der Ploeg M, Vooijs GP (1988) In situ hybridization as a tool to study numerical chromosome aberrations in solid bladder tumors. Histochemistry 89:307-316.

Kerstens HLM, Poddighe PJ, Hanselaar AGJM (1994) Double-target in situ hybridization in brightfield microscopy. J Histochem Cytochem 42:1071-1077.

Kerstens HJM, Poddighe PJ, Hanselaar AGJM (1995). A novel in situ hybridization signal amplification method, based on the deposition of biotinylated tyramine. J Histochem Cytochem 43:347-352.

Landegent JE, Jansen in de Wal N, Fisser-Groen YM, Bakker E, Ploeg van der M, Pearson PL (1986) Fine mapping of the Huntington disease linked D4S10 locus by non-radioactive in situ hybridization. Human Genet 73:354-357.

Little RD, Braaten DC (1989) Genomic organization of human 5S rDNA and sequence of one tandem repeat. Genomics 4:376-383.

Lomant AJ, Fairbanks G (1976) Chemical probes of extended biological structures: synthesis and properties of the cleavable protein cross-linking reagent [³⁵S]dithiobis(succinimidyl propionate). J Mol Biol 104:243-261.

Raap AK, Van de Corput MPC, Vervenne RAW, Van Gijlswijk RPM, Tanke JH, Wiegant J (1995) Ultra-sensitive FISH using peroxidase-mediated deposition of biotin- or fluorochrome tyramide. Hum Mol Genet 4:529-534.

Speel EJM, Kamps M, Bonnet J, Ramaekers FCS, Hopman AHN (1993) Multicolour preparations for in situ hybridization using precipitating enzyme cytochemistry in combination with reflection contrast microscopy. Histochemistry 100:357-366.

Speel EJM, Jansen MPHM, Ramaekers FCS, Hopman AHN (1994) A novel triple-color detection procedure for brightfield microscopy, combining in situ hybridization with immunocytochemistry. J Histochem Cytochem 42:1299-1307.

Speel EJM, Ramaekers FCS, Hopman AHN (1995) Cytochemical detection systems for in situ hybridization, and the combination with immunocytochemistry. "Who is still afraid of Red, Green and Blue?" Histochem J, 27:33-858.

Speel EJM, Ramaekers FCS, Hopman AHN (1997) Sensitive multicolor fluorescence in situ hybridization using catalyzed reporter deposition (CARD) amplification. J Histochem Cytochem 45:1439-1446.

Van Gijlswijk RPM, Wiegant J, Vervenne R, Lasan R, Tanke JH, Raap AK (1996). Horseradish peroxidase-labeled oligonucleotides and fluorescent tyramides for rapid detection of chromosome-specific repeat sequences. Cytogenet Cell Genet 75:258-262.

Van Gijlswijk RPM, Zijlmans HJMAA, Wiegant J, Bobrow MN, Erickson TJ, Adler KE, Tanke JH, Raap AK (1997) Fluorochrome-labeled tyramides: use in immunocytochemistry and fluorescence in situ hybridization. J Histochem Cytochem 45:375-382.

Van Heusden J, de Jong P, Ramaekers F, Bruwiere H, Borgers M, Smets G. (1997). Fluorescein-labeled tyramide strongly enhances the detection of low bromodeoxyuridine incorporation levels. J. Histochem Cytochem 45:315-319.

## Claims

1. A method of preparing a tyramide conjugate which comprises reacting a suitable ester of a labeling compound with tyramine or a salt thereof in a substantially water-free organic solvent at a constant pH of about 7 to about 9.5.

2. The method of Claim 1 wherein the pH is 7.0 to 8.0.

3. The method of Claim 1 or 2 wherein the reaction is carried out in a buffered medium.

4. The method of any one of the preceding claims wherein said ester of a labeling compound is the succinimidyl ester of a compound selected from the group of biotin, digoxigenin, dinitrobenzene, trinitrobenzene and the fluorochromes fluorescein, rhodamine, cyanine, coumarin and BODIPY dyes, and structurally closely related compounds.

5. A compound selected from the group consisting of digoxigenin-tyramide and 2,4,6-trinitrophenyl-tyramide.

6. Tyramide conjugate obtainable by the method claimed in any one of Claims 1-5.

7. Use of a tyramide conjugate according to any one of Claims 1-6, in the CARD amplification system or in an affinity-assay, including ELISA, immunoblotting, immunocytochemistry and ISH.

8. Affinity-assay, including ELISA, immunoblotting, immunocytochemistry and ISH, characterized in that it comprises a tyramide conjugate according to any one of Claims 1-6.

9. A method of preparing 2,4,6-trinitrophenyl-tyramide which comprises reacting an aqueous solution of the corresponding trinitrobenzenesulfonic acid with tyramine or a salt thereof at a pH of about 9.5.

10. The method of Claim 9 wherein the pH is adjusted by using an excess of triethylamine or by dissolving said tyramine or a salt thereof in an aqueous buffer system.
